(19) Europäisches Patentamt
European Patent Office
Office européen des brevets

(11) Publication number: **0 462 797 A2**

(12) **EUROPEAN PATENT APPLICATION**

(21) Application number: **91305498.7**

(22) Date of filing: **18.06.91**

(51) Int. Cl.[5]: **C07H 1/00**, C07H 1/06, C08B 37/00, // G01N35/00

(30) Priority: **21.06.90 GB 9013831**

(43) Date of publication of application: **27.12.91 Bulletin 91/52**

(84) Designated Contracting States: **DE ES FR GB IT**

(71) Applicant: **OXFORD GLYCOSYSTEMS LIMITED**
**Unit 4, Hitching Court, Blacklands Way**
**Abingdon, Oxon, OX14 1RG (GB)**

(72) Inventor: **Parekh, Rajesh Bhikhu**
**2 Pound Close**
**Kirtlington, Oxon OX5 3JR (GB)**
Inventor: **Merry, Anthony Hugh**
**22 Little Lees**
**Charlburry, Oxon OX7 3HB (GB)**
Inventor: **Bruce, James**
**53 Wytham View**
**Eynsham, Oxon OX8 1LY (GB)**

(74) Representative: **Courtney, Graham Sidney**
**Whitegates**
**East Hanney Wantage Oxon OX12 0HR (GB)**

(54) **Release and isolation of N-glycans.**

(57) The invention relates to a method for the release of N-glycan from a glycoconjugate and to isolation of such glycan.

The invention provides a method which includes subjecting a glycoconjugate to the influence of a hydrazine reagent, said glycoconjugate being substantially salt-free, and controlling conditions under which the glycoconjugate is subjected to the influence of the hydrazine reagent so as to cause release of N-glycan from the glycoconjugate in such a way as to allow subsequent recovery of N-glycan in substantially unreduced and intact form and in a high yield.

Released glycan may be isolated in accordance with the invention by use of a chromatographic material and, if required, performing an N-acetylation reaction and converting any acetohydrazone derivative to unreduced N-glycan.

EP 0 462 797 A2

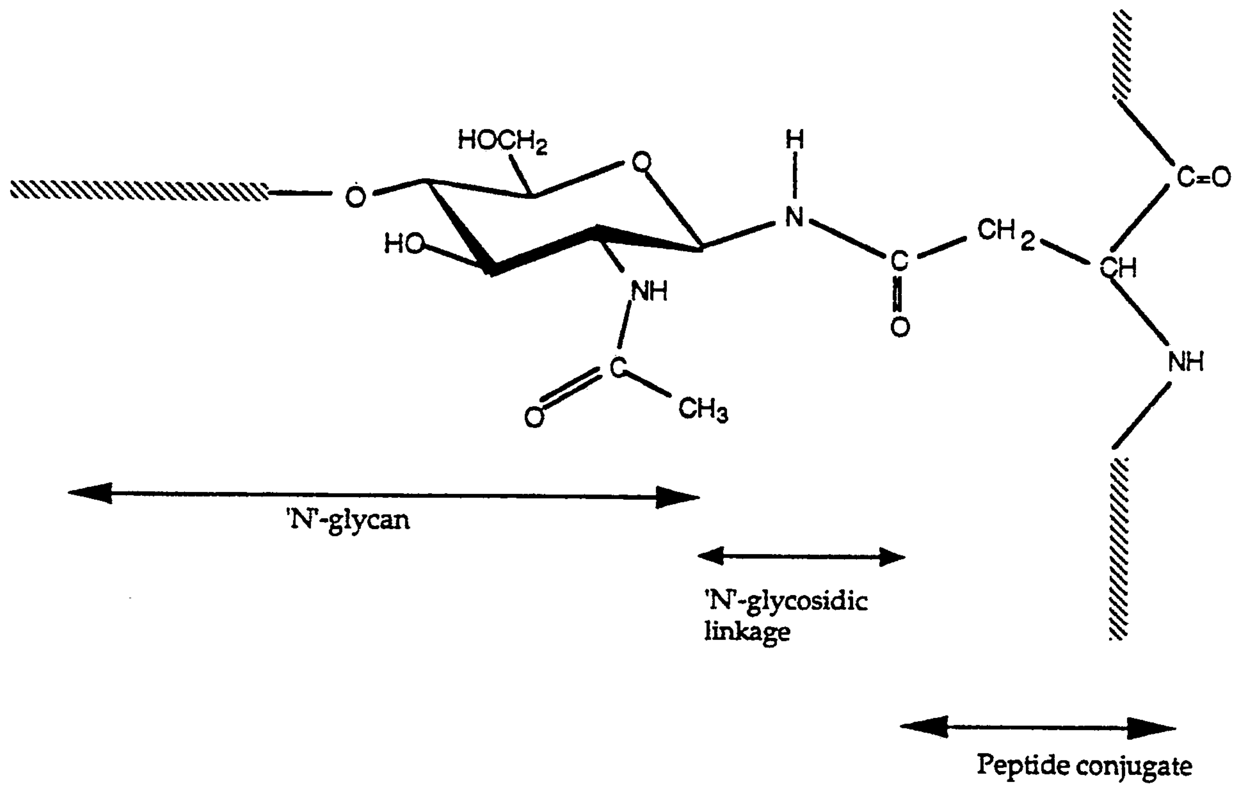


**Figure 1**

*Background of the Invention*

This invention relates to a method for the release of N-glycans (i.e. N-linked type oligosaccharides) from glycoconjugates and to isolation of such glycans.

Examples of glycoconjugates are glycoproteins, glycohormones and glycolipids.

An 'N-linked type' oligosaccharide is an oligosaccharide which is covalently bonded to a conjugate by an N-glycosidic linkage. An example of such a linkage as typically found on glycoproteins is shown in Figure 1 of the accompanying Drawings. For the purposes of this disclosure, release refers to an event or process leading to cleavage of the covalent N-glycosidic bond attaching an N-linked type oligosaccharide to a conjugate, and isolation refers to an event or process leading to physical separation of released oligosaccharides from conjugate or conjugate-derived materials.

The release and subsequent isolation of an N-linked oligosaccharide or N-linked type oligosaccharides (hereinafter referred to as N-glycan or N-glycans respectively) from a glycoconjugate are important for several reasons. Principal among these are the following: Firstly, a detailed structural characterisation of a glycoconjugate requires structural analysis of any such associated glycans. Since several such glycans may be attached to a single conjugate, and since structural analysis of glycans is most accurately performed on single, purified glycans, such an analysis requires prior release of such glycans from the conjugate, subsequent isolation of such glycans from the conjugate, and then purification of glycans from one another. Secondly, such glycans are increasingly recognised as important biological molecules in their own right. A study of the biological properties of N-glycans is preferentially undertaken using N-glycans free of the conjugate.

The criteria that ought to be simultaneously satisfied for any successful method for the release and isolation of an N-glycan from a glycoconjugate are as follows:

(i) The method of release should preferably cleave the N-glycosidic linkage in a manner independent of the nature of *both* the oligosaccharide component *and* the conjugate component.

(ii) The method of release should preferably achieve cleavage without permanent (i.e. not easily reversible) chemical damage to the cleaved glycans.

(iii) The method of isolation should preferably separate an N-glycan from a conjugate in a manner independent of the nature of *both* the oligosaccharide component *and* the conjugate component, as in (i), above.

(iv) The method of isolation should preferably achieve isolation without causing chemical damage to the cleaved glycans.

In addition, it is preferable if the method also achieves recovery in high yield (e.g. ≥85%) of released N-glycans, irrespective of the amount of starting glycoconjugate.

Previously, two different methods have been used for the release of N-glycans. These are briefly summarised below and assessed with respect to the above criteria.

A. *Enzymatic Methods* - For example, the use of proteolyticenzymes such as Pronase® to obtain glycopeptides from glycoproteins, and/or the use of a mixture of enzymes such as peptide N-glycosidase (E.C. number 3.2.2.18) to cleave some N-glycans from the conjugate. Such methods are generally unsatisfactory for several reasons, but principally because of the selectivity of glycan release. Enzymes are, by their very nature specific, and release only certain glycans and then in a manner influenced by the attached conjugate. That is, enzymatic methods as currently practised and understood, do not satisfy criterion (i), above.

B. *Chemical Methods* - Two chemical methods have been practised for the release of N-glycans. These are:

(i) *The use of alkaline solutions.* The N-glycosidic linkage attaching an N-glycan to a conjugate is labile to strong alkali at high temperatures, and alkaline solutions can therefore be employed for release of an N-glycan. For example, incubation with 1M NaOH at 100°C for *4* hours has been successfully employed. An established and accepted disadvantage of this method is the alkalilability of most N-glycans. Most N-glycans that are attached to a peptide are so attached through the structure shown in Figure 2 of the accompanying Drawings. Upon cleavage (Figure 2) by alkali, the reducing terminus monosaccharide (N-acetylglucosamine or GlcNAc in N-glycans) is further degraded by the alkali. Such degradation can be prevented by the reduction of the reducing terminus residue by performing the alkali-induced release in the presence of a vast excess of reducing agent. Typically 4M $NaBH_4$ is used. This reduction involves conversion of released N-glycan to the alditol form (Figure 2). Irrespective, therefore, of the presence or absence of excess reducing agent, the released N-glycan will be recovered in a permanently chemically altered form (i.e. not as the native compound). This method does not

therefore satisfy criterion (ii), above.

(ii) *The use of anhydrous hydrazine*. Numerous authors have studied the release by hydrazinolysis of N-glycans from glycoproteins. The currently preferred method is that of Rademacher and Dwek (European Patent Application No. 0 215 766A2). This is a method in which a set of conditions were defined allowing recovery of N-glycans in undefined yield, but intact with respect to structure. The method of Rademacher and Dwek was therefore an improvement on all previous methods, but as shown by the results disclosed in this specification, gives a relatively lower yield of N-glycans. Hence this method as currently practised is not generally applicable to the recovery of in high yield of N-glycans from a glycoprotein sample.

In summary, none of the above mentioned methods as currently practised and understood is suitable for the release of intact, unaltered, N-glycans in high yield.

*Brief Description of the Invention*

In accordance with one aspect of the present invention there is provided a method for releasing an N-glycan from a glycoconjugate which includes subjecting a glycoconjugate to the influence of a hydrazine reagent, said glycoconjugate being substantiallay salt-free, and controlling conditions under which the glycoconjugate is subjected to the influence of the hydrazine reagent so as to cause release of N-glycan from the glycoconjugate in such a way as to allow subsequent recovery of N-glycan in substantially unreduced and intact form and in a high yield.

In accordance with the present invention a high yield may be defined as ≧85%, but may be less than this as long as the yield is useful in practice.

The glycoconjugate optionally may be subjected to the influence of the hydrazine reagent and subjected to an energy input. Thus, for example, glycoconjugate and the hydrazine reagent may be brought together and the resulting reaction mixture heated by any suitable means. Other forms of energy input may be employed if desired (e.g. microwave radiation or infra-red radiation).

The hydrazine reagent may be hydrazine itself or any suitable derivative or compound thereof (e.g. related salt) capable of bringing about a desired cleavage of an N-glycosidic bond linking an N-glycan to a conjugate. The hydrazine reagent may be used, for example, in liquid form or in vapour form.

By way of example, the present invention provides a method for releasing unreduced intact N-glycans from glycoconjugates, which comprises heating a substantially salt-free and essentially anhydrous glyconjugate sample with principally anhydrous hydrazine reagent under optimal conditions so as to cause release from the glycoconjugate of N-glycan in such a way as to allow the subsequent recovery of such N-glycans in intact form.

The term "salt-free" as used in this Specification means not containing salt to an extent which leads to any unacceptable interference with the performance of a method in accordance with the present invention (e.g. release of an N-glycan from a glycoconjugate).

The term "anhydrous" as used in this Specification means not containing water to an extent which leads to any unacceptable interference with the performance of a method in accordance with the present invention (e.g. release of an N-glycan from a glycoconjugate).

The heating may be effected either microscopically or macroscopically.

In one embodiment the method of the present invention may include the step of removing salt from a glycoconjugate prior to subjecting it to the influence of a hydrazine reagent.

In another embodiment the method of the present invention may include the step of removing water from a glycoconjugate prior to subjecting it to the influence of a hydrazine reagent.

For the release of N-glycans it is not always necessary for the hydrazine reagent to be anhydrous, however where preferred the glycoconjugate may be subjected to the influence of the hydrazine reagent under substantially anhydrous conditions.

The invention also provides a method for the release of N-glycan from a glycoconjugate which method includes a step of isolating released intact and unreduced N-glycan.

Following release, it is preferable that any remaining, unreacted hydrazine reagent is removed to leave, as an unfractionated pool, the total population of N-glycans released.

It is also preferable that chromatographic procedures be performed to achieve substantial removal of conjugate or conjugate-derived material from N-glycans to leave N-glycans as an unfractionated pool free of such material.

With certain glycans, it may be necessary to perform an N-acetylation reaction involving N-acetylation of any of the free amino groups produced as a result of a reaction with hydrazine. Without prior knowledge of the full structure of glycans involved, it is difficult to predict in advance whether such N-acetylation will be necessary. It is therefore preferable to perform this reaction as a precautionary measure, in all cases.

It is also preferable that N-acetylated isolated glycans be subjected to acidic conditions (whether through the use of acid in immobilised form, mineral acid, or Lewis acid) to cleave any acetohydrazone derivatives that may form during the reaction, and so to regenerate the intact, unaltered N-glycans.

The present invention also provides a method for the release of an N-glycan which also includes isolation of an N-glycan which method includes the steps of subjecting a glycoconjugate to the influence of a hydrazine reagent, said glycoconjugate being substantially salt-free, controlling conditions under which the glycoconjugate is subjected to the influence of the hydrazine reagent so as to cause release of N-glycan from the glycoconjugate in such a way as to allow subsequent recovery of N-glycan in substantially unreduced and intact form, contacting the reaction mixture, formed by subjecting the glycoconjugate to the influence of the hydrazine reagent, with a first chromatographic material thereby to effect sorbing of N-glycan (and any derivative thereof) upon the chromatographic material, eluting the chromatographic material, said sorbing and said eluting being such as to effect separation of N-glycan (and any derivative thereof) from conjugate and/or conjugate-derived material and such as to remove any hydrazine reagent, and, if a derivative of an N-glycan is present which has free primary amino acid groups, performing an N-acetylation reaction to generate unreduced glycan from the derivative, and, if required by the manner in which any N-acetylation reaction was carried out, removing mono- and/or divalent cations from a reaction mixture obtained after the N-acetylation reaction, and separating from such a reaction mixture N-glycan, or a derivative thereof, by means of sorption upon a second chromatographic material and selective elution therefrom, and converting any derivative of the N-glycan to unreduced form.

By way of example, an N-acetylation reaction may be performed either before or after desorption from the first chromatographic material.

Also, by way of example, where any derivative of the N-glycan obtained after elution from the second chromatographic material is an acetohydrazone derivative mildly acidic conditions may be used to effect generation of unreduced N-glycan.

*Detailed Description of the Invention*

The invention can be applied to glyconjugates in general, however, by way of exemplification, reference will later be made to specific examples of glycoconjugates. One example of glycoconjugate is bovine fetuin, which is accepted in the scientific literature as a model glycoprotein for study, containing as it does, N-glycans.

Before subjecting the glycoconjugate to the influence of the hydrazine reagent (i.e. hydrazinolysis), the glycoconjugate and hydrazine reagent may be, for example, prepared as follows. The glycoconjugate is rendered essentially salt-free, for example by dialysis, gel filtration, or chromatography in a suitable system. Once salt-free, the glycoconjugate may be rendered essentially anhydrous, for example by lyophilisation, and the water content reduced to at least that achieved at equilibrium under lyophilisation conditions of 25 millibar at 25°C. The water content of the hydrazine reagent may be likewise relevant, and may be arranged so as not to exceed 4% volume/volume. Thus most commercially available samples of anhydrous hydrazine reagent are suitable, though drying of hydrazine reagent can be achieved through one of numerous reported processes. (However, in some circumstances a greater concentration of water may be present; for example, up to 6% water may be present in some applications of the present invention.) Suitable hydrazine reagent is then added to the appropriately prepared sample in an airtight vessel. The reaction between glycoconjugate and hydrazine reagent can be initiated by the input of energy, either microscopically or macroscopically, for example by raising the temperature. For any method of input of energy the optimal conditions for reaction can be deduced from various experimental approaches. In this disclosure the preferred method of initiating the reaction is through increase in temperature to a steady state.

By experimental measurement of yield at various temperatures over time it has been shown, in accordance with the present invention, that reaction of a glycoconjugate with a hydrazine reagent to release an N-glycan follows first-order reaction kinetics.

The preferred theoretical framework therefore for analysis of experimental results is the form of the Arrhenius equation which is commonly used to define to a first approximation the dependence of the rate of a reaction on temperature. This form is as follows:

$$k = Ae^{-Eact/RT}$$

where

| | |
|---|---|
| k | = reaction rate |
| A | = Arrhenius constant |
| R | = Universal gas constant |
| Eact | = Activation energy |
| T | = temperature |

then subject to an additional degradative reaction, it is deduced from the Arrhenius equation that:

mole fraction of N-glycans released in intact form =

$$m_N = -\left[e^{-(A_{rN}e^{-E_N^A/RT})\cdot t}\right] + \left[e^{-(A_{DN}e^{-E_N^D/RT})\cdot t}\right]$$

where

e = natural antilogarithm

$A_{rN}$ = Arrhenius constant for the release of N-glycans

$A_{DN}$ = Arrhenius constant for the degradation of N-glycans

$E^A_N$ = Activation energy for the release of N-glycans

$E^D_N$ = Activation energy for the degradation of N-glycans

R = Universal gas constant

T = Temperature

t = time

Clearly, for recovery in high yield of N-glycans, (T,t) may be chosen so that m ≧0.85 (corresponding to ≧85% recovery)

Conditions may be selected in accordance with the above equation such as to achieve a preselected yield of intact N-glycan (e.g. a yield such as ≧85%).

Although a preferred yield is a high yield such as ≧85% there may be applications where lower yields are acceptable and conditions which give rise to such lower yields may be selected in accordance with the above equation.

Conditions of temperature and time suitable for release of intact N-glycans in a high yield can be deduced from experiments in which the release of intact N-glycans is measured as a function of temperature only, since such experiments allow a determination of $A_{rN}$, $A_{dN}$, $E^A_N$ and $E^D_N$. The results of such an experiment are shown in Figure 3 of the accompanying Drawings in which the relative release of intact N-glycans is measured after incubation of the glycoprotein bovine fetuin with suitable hydrazine for 8 hours at various temperatures.

From the data in Figure 3, the approximate conditions of temperature and time required for any given percentage recovery of intact N-glycans can be calculated. One such set of conditions, yielding N-glycans in ≥85% yield, and used in this disclosure is 100° Celsius for 10 hours. Similar analyses can be used to define reaction conditions for other forms of initiation of the reaction.

At this point the reaction to achieve successfully the release of N-glycans from glycoconjugate is complete. For further study and analysis of released glycans it is preferable that the glycans be first isolated from unreacted hydrazine reagent and conjugate or conjugate-derived material. After the incubation, unreacted hydrazine reagent can be removed in one of two ways. Firstly, by evaporation under reduced pressure, followed by repetitive co-evaporation with a miscible agent (such as anhydrous toluene). Secondly, and the preferred method as described in this Specification, by the adsorption of the glycans in the reaction mixture to a chromatographic medium, of which the preferred medium in this Specification is a cellulose-based medium, so as to remove unreacted hydrazine reagent and also substantially remove conjugate or conjugate-derived material from released glycans without repeated manipulation of the glycans, without selective loss of yield of released glycans, and without the introduction of contaminant material. This is performed as follows:

To a column of microcrystalline cellulose (a commercially available example of which is Avicel®) equilibrated in a suitable extraction solvent an example of which is butanol, ethanol, and acetic acid (varying between 4:1:0.01-0.6 (v/v/v)), the sample is applied. The N-glycans and some contaminants are adsorbed to the cellulose particles, and the hydrazine reagent eluted away using the extraction solvent (typically three column washes of solvent are required), and the N-glycans desorbed (and so eluted) with the use of methanol and water or desired aqueous buffer (typically 2-3 column volumes).

The N-glycans so obtained may contain a proportion of free primary amino groups. These groups can therefore be N-acetylated, if desired, to generate the authentic unreduced N-glycans. Numerous methods have been reported for performing such N-acetylation. In a preferred embodiment, the N-glycans are N-acetylated by reaction with excess acetic anhydride in either the methanol/water mixture or aqueous sodium acetate buffer solution, pH 5.0, or IM sodium bicarbonate solution. Such N-acetylation can be performed at any temperature between 0°C and 37°C with no loss of efficiency and quantitative N-acetylation occurs in ~ 30 minutes. The use of methanol/water or sodium acetate solution are preferred since either can be used to elute the N-glycans from the cellulose column used for hydrazine reagent removal, thus avoiding any manipulation of the sample prior to N-acetylation. In those instances where aqueous buffer containing monovalent cations are used, such

cations are removed by passage of the N-acetylation mixture through a strong cation exchange resin in the proton form. Numerous such resins are available and suitable, but a preferred resin is Dowex AG50X12 ($H^+$), a styrene/divinyl benzene polymeric lattice carrying sulphonic acid functional groups. In those instances where aqueous buffer containing mono- and/or di-valent cations are used, a mixed-bed column containing sufficient resins to remove both such ions is used. This would typically require the use of Chelex 100 ($Na^+$) to remove divalent cations, and Dowex AG50X12 ($H^+$) to remove monovalent cations. In those instances where N-acetylation is performed in the absence of cations, no such cation exchange chromatography is required.

At this stage in the method, any remaining traces of conjugate or conjugate-derived materials are removed from the N-acetylated mixture containing the glycans, by a chromatography process. To a column of microcrystalline cellulose previously washed sequentially with water and methanol, and then extensively equilibrated in butanol, ethanol, water solvent (or composition 4:1≦0.5), the sample free of cations is applied in a solvent of composition butanol, ethanol, water of 4:1:≦0.5 (v/v/v). After loading all the sample, any conjugate or conjugate-derived material is eluted away from the adsorbed glycans by use of a butanol, ethanol, water solvent (of typical composition 4:1:≧0.5, v/v/v), or a toluene, methanol, acetic acid solvent (of typical composition 3:1:1, v/v/v). Typically, 3-5 column volumes of wash are required to assure successful removal of such material. The N-glycans are then desorbed by elution with methanol, water, or a desired aqueous buffer, and the N-glycans so eluted are collected.

At this stage of the method, a solution exists containing the intact N-glycans (originally attached to glycoconjugate), in a solvent of composition related to that used to elute the N-glycans from the cellulose column, above. The N-glycans at this stage consist of a mixture of authentic, unreduced, oligosaccharides and the acetohydrazone derivatives of such N-glycans. Conversion of acetohydrazone derivatives to the authentic unreduced N-glycans by cleavage of the acetohydrazone is achieved by exposing the glycan mixture to mild acid, whether immobilised, mineral or Lewis acid. Various such methods have been previously reported, and are generally satisfactory. The N-glycans are generally removed from solvent by evaporation, resuspended in water, and lyophilised prior to further study.

By the above method, intact, unreduced N-glycans are obtained free of contaminating conjugate or conjugate-derived material in high yield from glycoconjugate containing N-glycans attached via N-glycosidic linkages to the conjugate. The presence of conjugate or conjugate-derived material in the final pool can be assessed by a variety of techniques, of which one such is acidic hydrolysis (6N HCl, 104°C, 60 minutes), followed by ninhydrin-based quantitation of peptide material; this is a standard procedure available to one skilled in the art.

The integrity and quantity of the N-glycans can be assessed by numerous techniques. Two examples which may be used in relation to the present invention are:

(1) Composition analysis of the monosaccharides obtained from the N-glycans.

(2) High resolution gel permeation chromatography of the reduced radiolabelled (tritiated) alditols of the N-glycans.

The present invention is described, by way of example only, with reference to the accompanying Drawings and with reference to Examples 1 to 4.

The Drawings show:

Figure 1: Drawing of the N-glycosidic linkage attaching an N-glycan to a peptide.

Figure 2: Drawing summarising the significant chemical reactions in the currently practised method for cleaving N-glycosidic bond by the use of alkaline conditions.

Figure 3: A graph summarising the results of a study conducted to measure the effect of temperature of incubation on release of N-glycans from the glycoprotein bovine fetuin using an hydrazinolysis reaction.

Figure 4A: Size chromatogram of glycan alditols derived from bovine berum fetuin.

Figure 4B: Size chromatogram of glycan alditols derived from human serum α1-acid glycoprotein.

Figure 4C: Size chromatogram of alditols derived from horseradish peroxidase.

Figure 4D: Size chromatogram of alditols derived from hen egg ovalbumin.

Figure 5: A schematic diagram of an instrument shown to perform the release and/or isolation of unreduced N-glycans from a glycoconjugate in an automated fashion.

*Example 1: Release and Isolation of N-glycans from Bovine Fetuin*

In this Example N-glycans were released from the glycoprotein bovine fetuin and the N-glycans so recovered assessed with respect to integrity, yield and purity.

Fetuin from foetal calf serum was purchased from the Sigma Chemical Company, (Poole, Dorset, UK). 1 milligramme of the glycoprotein was rendered salt-free by exhaustive microflow dialysis against glass-distilled water. After dialysis, the solution was lyophilised in a clean glass reaction tube using an Edwards Modulyo freeze-drier operating at 25 millibar vacuum. To the lyophilised protein was added 0.5ml of anydrous hydrazine

(of relative water content ~2.0% v/v). The tube was sealed under an anhydrous and oxygen-free atmosphere at room temperature and then placed in an oven equilibrated at 100°C for 10 hours.

After this time, unreacted hydrazine was removed from the reaction mixture in the tube by column chromatography as follows: to the top of a pre-prepared, pre-equilibrated (in butanol, ethanol, acetic acid solvent of composition 4:1:0.15, v/v/v) column of microcrystalline cellulose (Avicel®), was added 4ml of equilibration solvent without flow. The reaction mixture from the tube (i.e. the hydrazinolysate) (0.5ml) was then added to the solvent, followed by thorough mixing to create a single-phase, all without flow. Liquid flow was then begun, and when all liquid had passed through the column, the column was washed with 3 column volumes of equilibration solvent. The column was next washed with 1 column volume of methanol, and then two column volumes of aqueous sodium acetate (200 mM, pH 5.0). Re-N-acetylation was performed by the addition of 0.5ml acetic anhydride to pooled elutant and incubation at room temperature for 1 hour.

The resulting N-acetylation mixture was then passed through a Dowex AG50X12 ($H^+$) column of volume 2.2ml, and the column was then washed with five column volumes of distilled water. The elutant and washings were combined and then rotary evaporated to dryness. The entire sample was then taken up in 0.3ml glass distilled water and applied to the top of a column of pre-prepared, pre-equilibrated microcrystalline cellulose (0.8cm x 5cm) containing 2.0ml of butanol, methanol (4:1, v/v) above the cellulose bed without flow. The aqueous phase and the butanol/ethanol phase were mixed thoroughly into a single homogeneous phase, and flow was then begun. After all the'liquid had passed through, the column was washed with 5 column volumes of butanol, ethanol, water solvent (of composition 4:1:0.5, v/v/v). The column was next washed with 1 column volume of methanol and 2 column volumes of distilled water. The methanol and water fractions were co-pooled, rotary evaporated, taken up in 0.2ml of 1mM aqueous copper (II) acetate solution, incubated at room temperature for 30 minutes, and passed through a tandem column of Chelex 100 ($Na^+$) and Dowex AG50X12 ($H^+$) containing 300µl of each resin. The column was washed with five column volumes of water and the eluant and washings co-pooled, filtered (0.2µM Teflon membrane filter), rotary evaporated to dryness, and taken up in 1ml of glass distilled water. An aliquot of the unreduced oligosaccharides was analysed for monosaccharide composition and amino acid content by the ninhydrin method. (The results are given in Table I below.) Another aliquot of the unreduced oligosaccharides was radiolabelled by reduction in alkaline $NaB^3H_4$ and the radiolabelled oligosaccharide alditols so obtained were deacidified and analysed with respect to size. (The results are given in Figure 4A of the accompanying drawings.)

*Example 2: Release and Isolation of N-glycans from Human Serum α1-acid glycoprotein*

In this Example essentially the same procedure was followed as given in Example 1 with the exception that the glycoconjugate was human serum α1-acid glycoprotein not bovine fetuin.

Results of analyses for monosaccharide composition and amino acid content are given in Table I below.

The results of size analysis are given in Figure 4B of the accompanying Drawings.

*Example 3: Release and Isolation of N-glycans from Horseradish Peroxidase*

In this Example essentially the same procedure was followed as given in Example 1 with the exception that the glycoconjugate was horseradish peroxidase and not bovine fetuin.

Results of analyses for monosaccharide composition and amino acid content are given in Table I below.

The results of size analysis are given in Figure 4C of the accompanying Drawings.

*Example 4: Release and Isolation of N-glycans from Hen Egg Ovalbumin*

In this Example essentially the same procedure was followed as given in Example 1 with the exception that the glycoconjugate was hen egg ovalbumin.

Results of analyses for monosaccharide composition and amino acid content are given in Table I below.

The results of size analysis are given in Figure 4D of the accompanying Drawings.

The overall conclusion of these analyses is that contaminants other than peptide material are not detectable, that the level of peptide is <10% (by weight), and that N-glycans are intact, and recovered in high (e.g. ≧85%) yield.

Table I

| | Total content of N-acetyl-glucosamine (nanomoles)* | Total content of amino acids (μgrams) |
|---|---|---|
| Starting bovine fetuin | 319 | 1000 |
| Bovine fetuin N-glycan pool | 284 | 47 |
| Starting human serum α1-acid | 801 | 1000 |
| Human serum α1-acid N-glycan pool | 746 | 53 |
| Starting horseradish peroxidase | 246 | 1000 |
| Horseradish peroxidase N-glycan pool | 230 | 61 |
| Starting hen egg ovalbumin | 192 | 1000 |
| Hen egg ovalbumin N-glycan pool | 171 | 39 |

*It is generally accepted that the content of N-glycans is directly related to the content of N-acetylglucosamine. The content of N-acetylglucosamine is therefore used as a preferred measure of the N-glycan content in both the starting glycoconjugate and the glycan pool obtained after performing the methods described.

By treating the above glycoconjugates in accordance with the present invention, it has been established that certain sequences of processing events allow recovery with high yield (e.g. ≧85%), of intact N-glycans free of contaminants. A sequence of events has been defined by which intact N-glycans can be so recovered, and this sequence is such as to be readily automated. That this is so is proven by the construction and successful functioning of a machine which is able to receive a lyophilised sample of glycoconjugate and successfully deliver the intact N-glycans previously associated with that glycoconjugate.

*Description of the automated process*

N-linked glycans can be released from a glycoconjugate using the method of the present disclosure with the automated process shown in Figure 5.

Note that all the vessels in the upper row, which contain respectively water, methanol, butanol/ethanol 4:1 (v/v), butanol/ethanol/acetic acid 4:1:0.15 (v/v/v), 0.2M sodium acetate solution, 0.1M copper acetate/acetic acid pH 4.0 solution, acetic anhydride (17.4M), and hydrazineare connected to a supply of anhydrous argon of commercially available grade. Desired aliquots of the contents of each vessel can be supplied along path

L1 by pressurising the relevant vessel(s) with argon through the use of the pressurised argon input line, and such delivery is under the control of the system software. In the entire process described in this Specification, all solvents and reagents are stored in, and delivered from, one of the above vessels.

The glycoconjugate under investigation is rendered substantially salt-free, for example by dialysis, gel filtration, or chromatography in a suitable system. Once salt-free, the glycoconjugate is rendered essentially anhydrous, for example by lyophilisation and the water content of the sample reduced to at least that achieved at equilibrium under lyophilisation conditions of 25 millibar at 25°C. The essentially anhydrous sample is put into Reactor R1 which is then connected into the automated system as shown in Figure 5 of the accompanying Drawings. Prior to hydrazinolysis of the sample, anhydrous argon is first passed over the sample along Path L1, L3 to substantially replace the air in Reactor R1 with argon. Hydrazine of relative water content ≦2.0% (v/v) is then delivered to Reactor R1 along path L1, L3 from the hydrazine containing vessel shown connected to path L1 prior to point C1, as shown in the upper part of the Figure 5.

**Release of N-glycans**

Following delivery of the aliquot of hydrazine to R1, the hydrazinolysis reaction leading to release of N-linked glycans is performed by raising the temperature in Reactor R1 (through the use of a surrounding heating element) for a time selected to accord with the present invention; typically 100°C for 10 hours, at which point the reaction mixture is allowed to cool towards ambient temperature.

**Isolation of N-glycans**

Hydrazine Removal from Released Glycans

In order to remove any unreacted hydrazine by a chromatographic process the contents of the Reactor R1 is transferred in aliquots under argon pressure along path L3, L5, L2 to chromatography column A (containing a cellulose-based medium, such as microcrystalline cellulose) and during this latter passage, is mixed with a solvent of butanol, ethanol and acetic acid 4:1:0.15 (v/v/v), (delivered along path L1), using valves V1 and V2, at an approximate ratio of 5% contents of R1 to 95% solvent (v/v), and the mixture is passed through column A to waste W2. A further 3ml of the solvent of composition butanol, ethanol and acetic acid 4:1:0.15 (v/v/v) is then delivered to the Reactor R1 along path L1, L3, and from there to column A along path L3, L5, L2 with the column outlet again directed to waste W2. This is repeated with 6ml of the same solvent to ensure that all traces of hydrazine are removed from the Reactor R1. A volume of 6ml of the solvent of composition butanol, ethanol 4:1 (v/v) is delivered to the Reactor R1 along path L1, L3 and from there to column A along path L3, L5, L2 to remove any acetic acid present in column A, with the outlet of column A still connected to waste W2.

N-acetylation of any de-N-acetylated Glycans

To perform the re-N-acetylation, 0.2ml of acetic anhydride is delivered to the Reactor R1 along path L1, L3, and then to column A along path L3, L5 and L2 and during this latter passage, it is mixed with the solvent butanol, ethanol 4:1 (v/v) (delivered along path L1), using valves V1 and V2, at an approximate ratio of 5% acetic anhydride to 95% solvent (v/v), and the mixture is passed through column A to waste W2. This delivery, and the N-acetylation reaction is continued for 30 minutes. At the end of this period, to remove excess acetic anhydride, 3ml of the solvent butanol, ethanol 4:1 (v/v) is delivered to column A along path L1, L2. In all operations the outlet from Column A is directed to waste W2. At the end of this step, N-glycans and conjugate or conjugate-derived material are adsorbed to the cellulose of column A.

Separation of Glycans from Conjugate Material

In order to remove conjugate or conjugate-derived material a volume of 0.5ml water is delivered to the Reactor R1 via path L1, L3. The contents of R1 are then mixed with the solvent butanol, ethanol 4:1 (v/v), (delivered along path L1), by using valves V1 and V2, at an approximate ratio of 5% water to 95% solvent (v/v) to form a solvent mixture of composition butanol, ethanol and water 4:1:0.25 (v/v/v), and this solvent is passed through column A, with the outlet of column A connected to waste W2.

Desorption of the N-glycans from the cellulose of column A then takes place as follows. Methanol, (0.2ml) is passed along path L1, L2, through the column A, in 4 pulses (of 50μl aliquots each), each pulse being immediately followed with an argon pulse, with the outlet of column A connected to the holding vessel (H1) by path L4. The sodium acetate solution (3.0ml) is then passed to column A with similar pulses of argon along

path L1, L2, with the outlet of column A still connected to the holding vessel H1.

To ensure complete re-N-acetylation of the N-glycans a volume of acetic anhydride (0.1ml) is then passed along path L1, L3, L4 to the holding vessel H1, and the reaction mixture left for 30 minutes at ambient temperature.

Regeneration of Intact, Unaltered Glycans

To replace $Na^+$ ions with $H^+$ ions, the contents of the holding vessel (H1) are then passed along path L4 through the chromatography column B, containing a bed of 1.0ml Dowex Ag50X12 ($H^+$), and through column B directly to the Reactor R1 through path L2, L5, L3. To achieve glycan-acetohydrazone cleavage, and so regenerate intact and unaltered glycans, a solution of 0.2ml of copper acetate is then delivered to the Reactor R1 through path L1, L3, and the reaction mixture left for 60 minutes at ambient temperature. To replace $Cu^{2+}$ ions with $H^+$ ions,0 the mixture is then passed from R1 through the chromatography column C containing a mixture of 0.5ml of each of Chelex 100 ($Na^+$) and of Dowex AG50X12 ($H^+$), along path L3, L5, L2, to the remote collection port through path L4, L9. A volume of 1ml of water is then delivered to the Reactor R1 along path L1, L3 for washing purposes, and then through column C to the remote collection port through path L3, L5, L2, L4, L9. The solutions received at the remote collection port contain the released intact N- and O-glycans in a solution of dilute acetic acid, and may be removed from the instrument for further analysis.

## Claims

1. A method for releasing an N-glycan from a glycoconjugate characterised in that the method includes subjecting a glycoconjugate to the influence of a hydrazine reagent, said glycoconjugate being substantially salt-free, and controlling conditions under which the glycoconjugate is subjected to the influence of the hydrazine reagent so as to cause release of N-glycan from the glycoconjugate in such a way as to allow subsequent recovery of N-glycan in substantially unreduced and intact form and in a high yield.

2. A method as claimed in Claim 1 wherein the glycoconjugate and hydrazine reagent are substantially anhydrous.

3. A method as claimed in Claim 1 or Claim 2 wherein the yield of N-glycan is ≧85%.

4. A method as claimed in any one of Claims 1 to 3 for releasing unreduced and intact N-glycans from glycoconjugates comprising heating a substantially salt-free glycoconjugate with hydrazine reagent under optimal conditions so as to cause release from the conjugate of N-glycan in such a way as to allow the subsequent recovery of N-glycan in intact form.

5. A method as claimed in any one of Claims 1 to 4 for the release of an N-glycan which also includes isolation of an N-glycan which method includes the steps of subjecting a glycoconjugate to the influence of a hydrazine reagent, said glycoconjugate being substantially salt-free, controlling conditions under which the glycoconjugate is subjected to the influence of the hydrazine reagent so as to cause release of N-glycan from the glycoconjugate in such a way as to a llow subsequent recovery of N-glycan in substantially unreduced and intact form, contacting the reaction mixture, formed by subjecting the glycoconjugate to the influence of the hydrazine reagent, with a first chromatographic material thereby to effect sorbing of N-glycan (and any derivative thereof) upon the chromatographic material, eluting the chromatographic material, said sorbing and said eluting being such as to effect separation of N-glycan (and any derivative thereof) from conjugate and/or conjugate-derived material and such as to remove any hydrazine reagent, and, if a derivative of an N-glycan is present which has free primary amino acid groups, performing an N-acetylation reaction to generate unreduced glycan from the derivative, and, if required by the manner in which any N-acetylation reaction was carried out, removing mono- and/or di-valent cations from a reaction mixture obtained after the N-acetylation reaction, and separating from such a reaction mixture N-glycan, or a derivative thereof, by means of sorption upon a second chromatographic material and selective elution therefrom, and converting any derivative of the N-glycan to unreduced form.

6. A method for the release of an N-glycan as claimed in any one of Claims 1 to 5 wherein conditions are selected in accordance with the following equation:

$$m_N = -\left[e^{-(A_{rN}e^{-E_N^A/RT})\cdot t}\right] + \left[e^{-(A_{DN}e^{-E_N^D/RT})\cdot t}\right]$$

where

| | |
|---|---|
| $m_N$ | = mole fraction of N-glycan released in intact form |
| e | = natural antilogarithm |
| $A_{rN}$ | = Arrhenius constant for the release of N-glycans |
| $A_{DN}$ | = Arrhenius constant for the degradation of N-glycans |
| $E^A{}_N$ | = Activation energy for the release of N-glycans $E^A{}_N$ = Activation energy for the degradation of N-glycans |
| R | = Universal gas constant |
| T | = Temperature |
| t | = time |

such as to achieve a preselected yield of intact N-glycan.

7. A method as claimed in Claim 6 wherein the conditions are selected to achieve ≧85% yield of intact N-glycan.

8. A method as claimed in any one of Claims 1 to 7 wherein the glycoconjugate is a glycoprotein, a glycohormone, or a glycolipid.

9. A method as claimed in any one of Claims 1 to 8 wherein the hydrazine reagent is hydrazine, hydrazine vapour, a compound of hydrazine or a derivative of hydrazine.

10. A method as claimed in Claim 5 wherein an N-acetylation reaction is performed before desorption from the first chromatographic material.

11. A method as claimed in Claim 5 wherein an N-acetylation reaction is performed after desorption from the first chromatographic material.

12. A method as claimed in any one of Claims 1 to 11 wherein any derivative of the N-glycan obtained which derivative is an acetohydrazone derivative is subjected to acidic conditions to effect generation of unreduced N-glycan.

13. A method as claimed in any one of Claims 1 to 12 wherein the hydrazine reagent has a water content not exceeding 4% volume/volume.

14. A method as claimed in any one of Claims 1 to 13 wherein the glycoconjugate is bovine serum fetuin, human serum α1-acid glycoprotein, horseradish peroxidase or hen egg ovalbumin.

15. A method as claimed in any one of Claims 1 to 14 wherein an intact N-glycan is released from a glycoconjugate by incubation with hydrazine at 100°C for 10 hours.

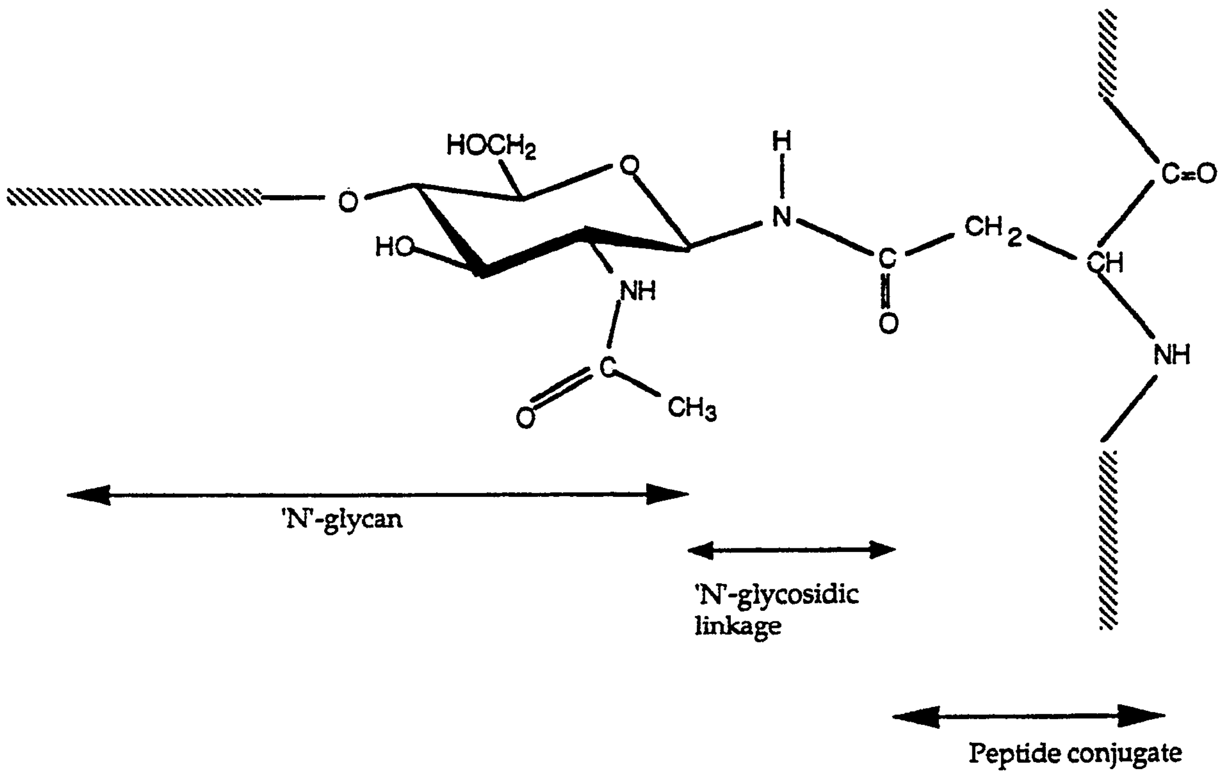


Figure 1

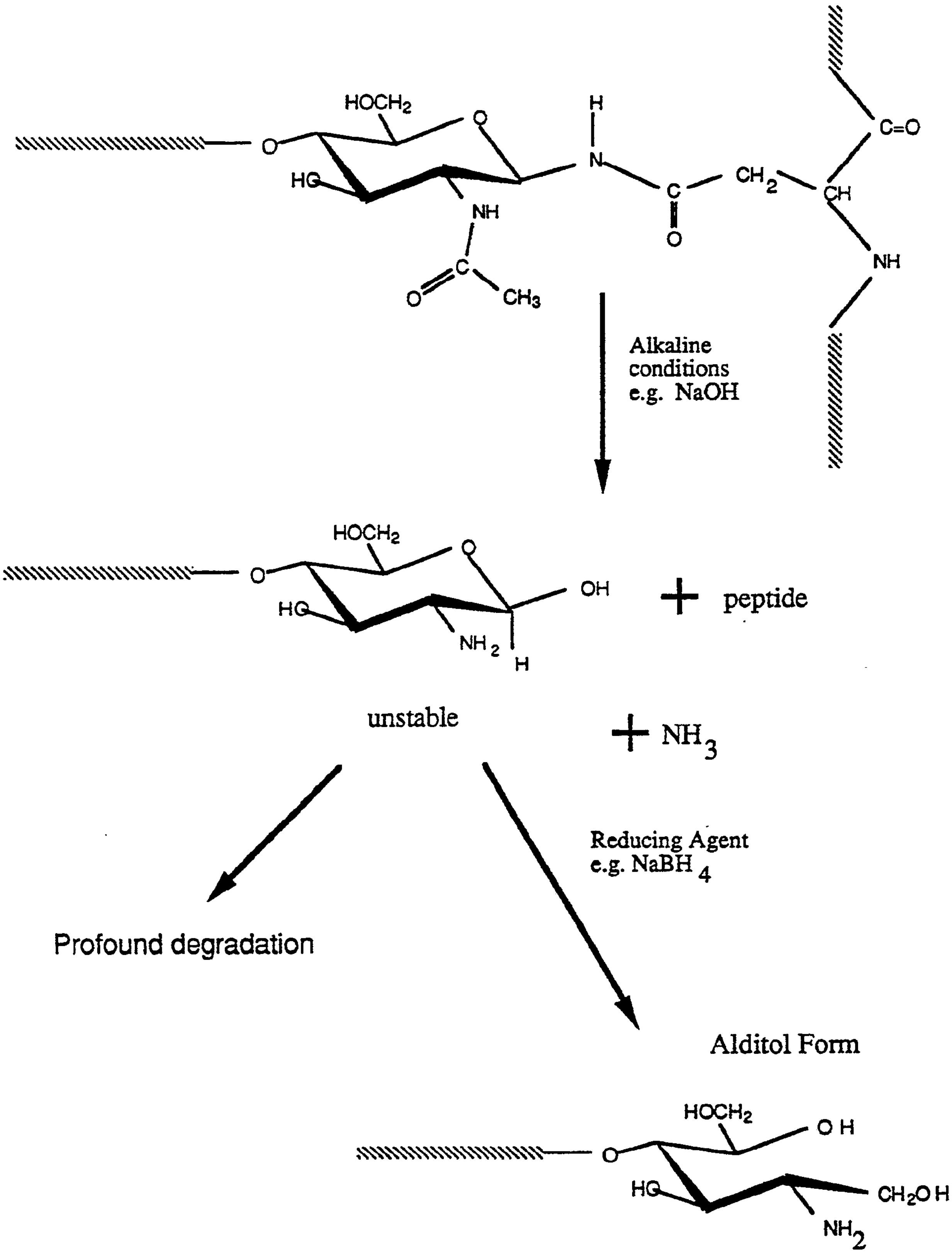
Alkaline conditions e.g. NaOH
+ peptide
unstable
+ $NH_3$
Reducing Agent e.g. $NaBH_4$
Profound degradation
Alditol Form

Figure 2

Oxford GlycoSystems

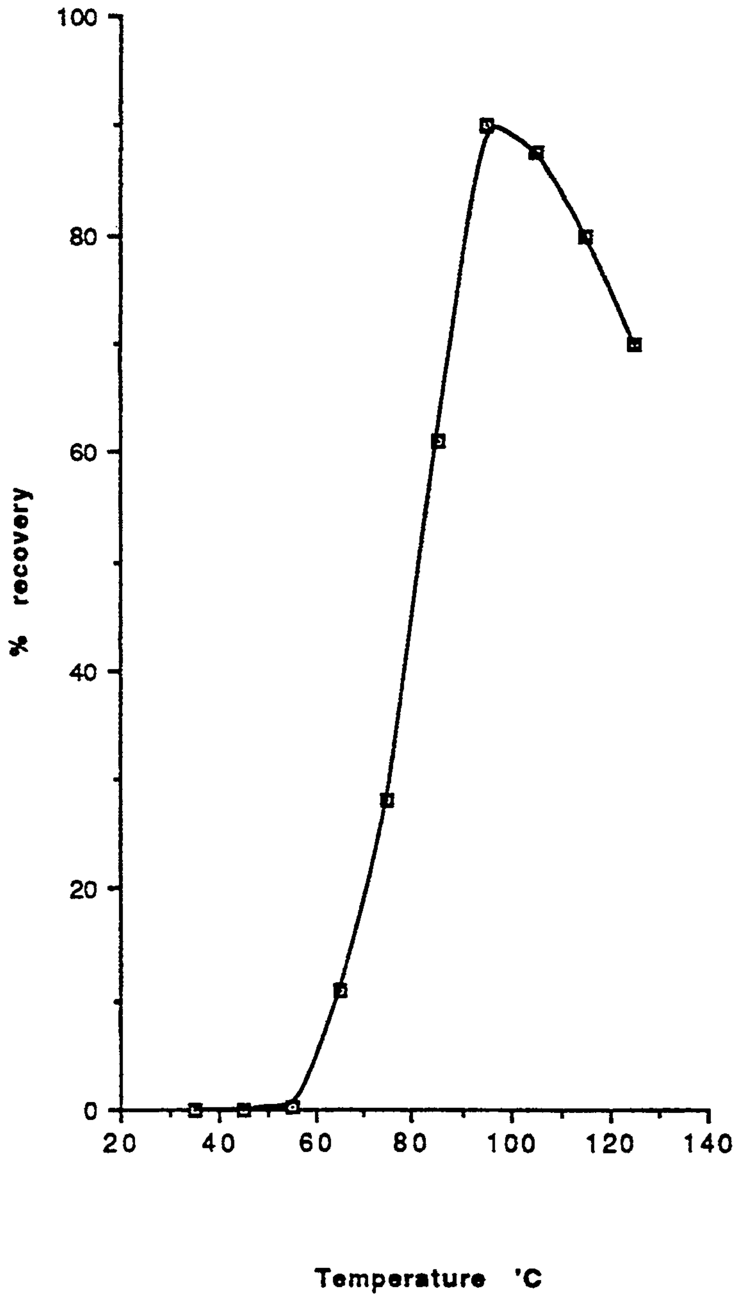


Figure 3

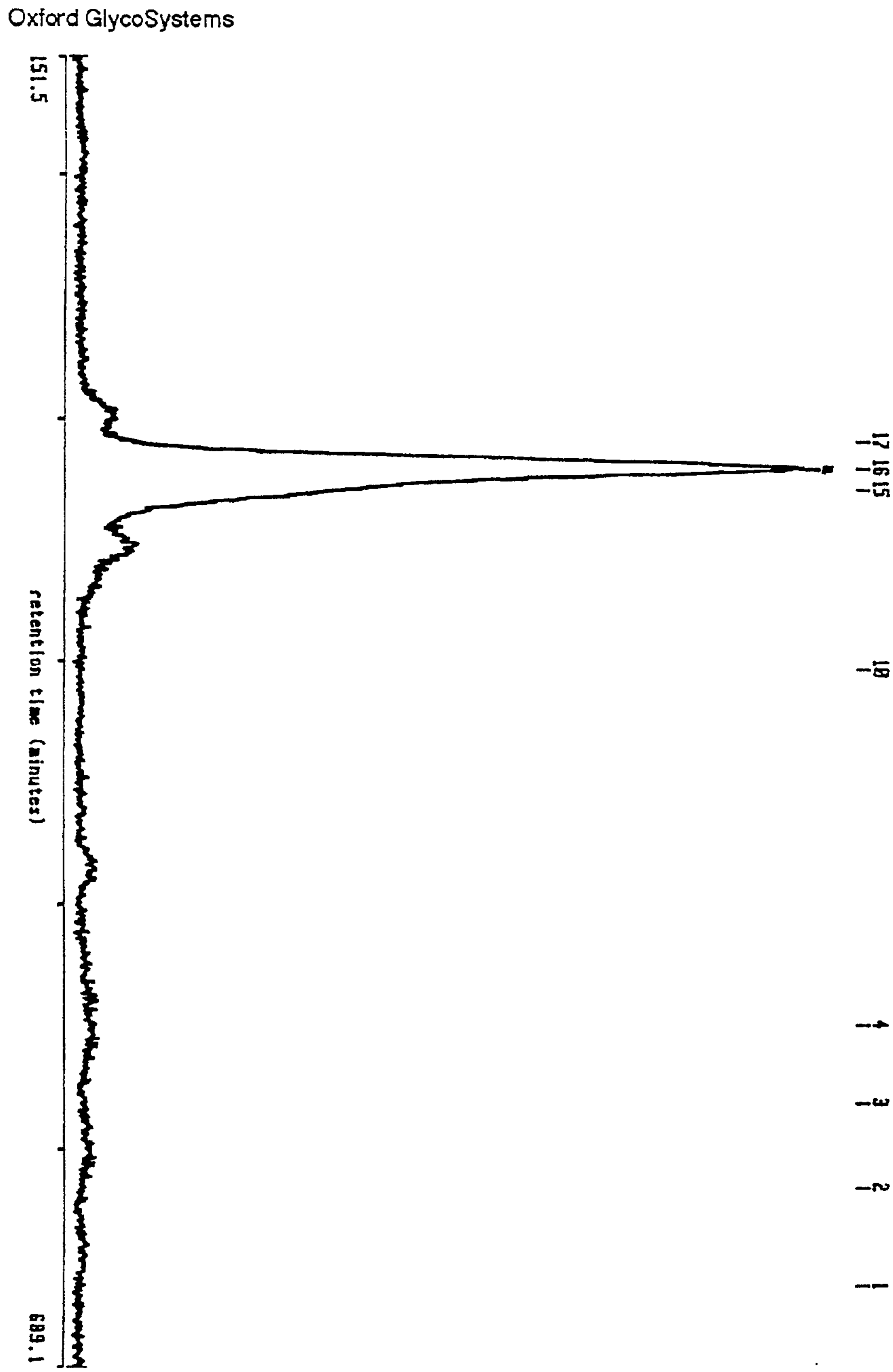
Oxford GlycoSystems
151.5
689.1
retention time (minutes)
17 16 15
10
4
3
2
1

Figure 4

Figure 4B

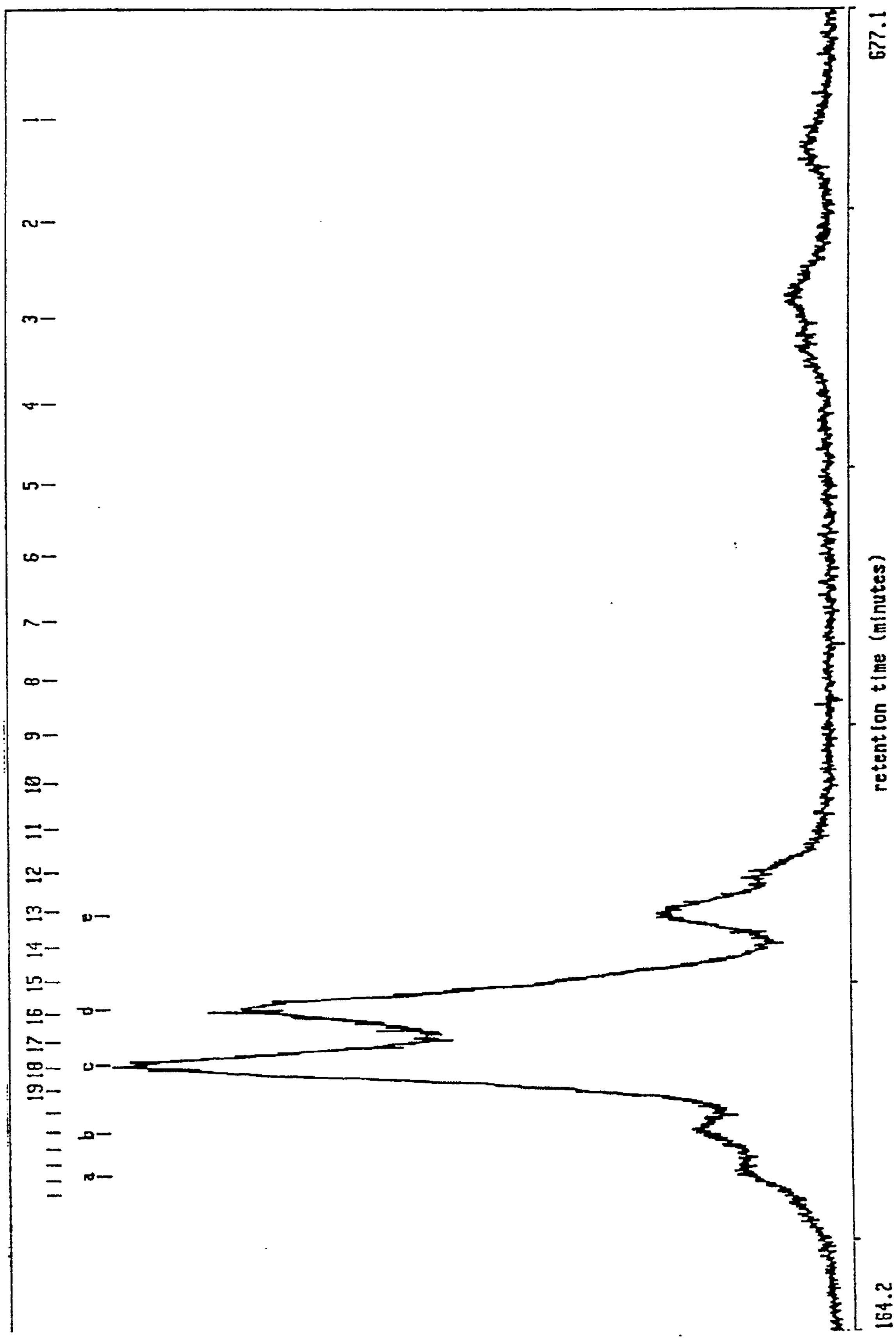
retention time (minutes)
164.2
677.1
a
b
c
d
e
1
2
3
4
5
6
7
8
9
10
11
12
13
14
15
16
17
18
19

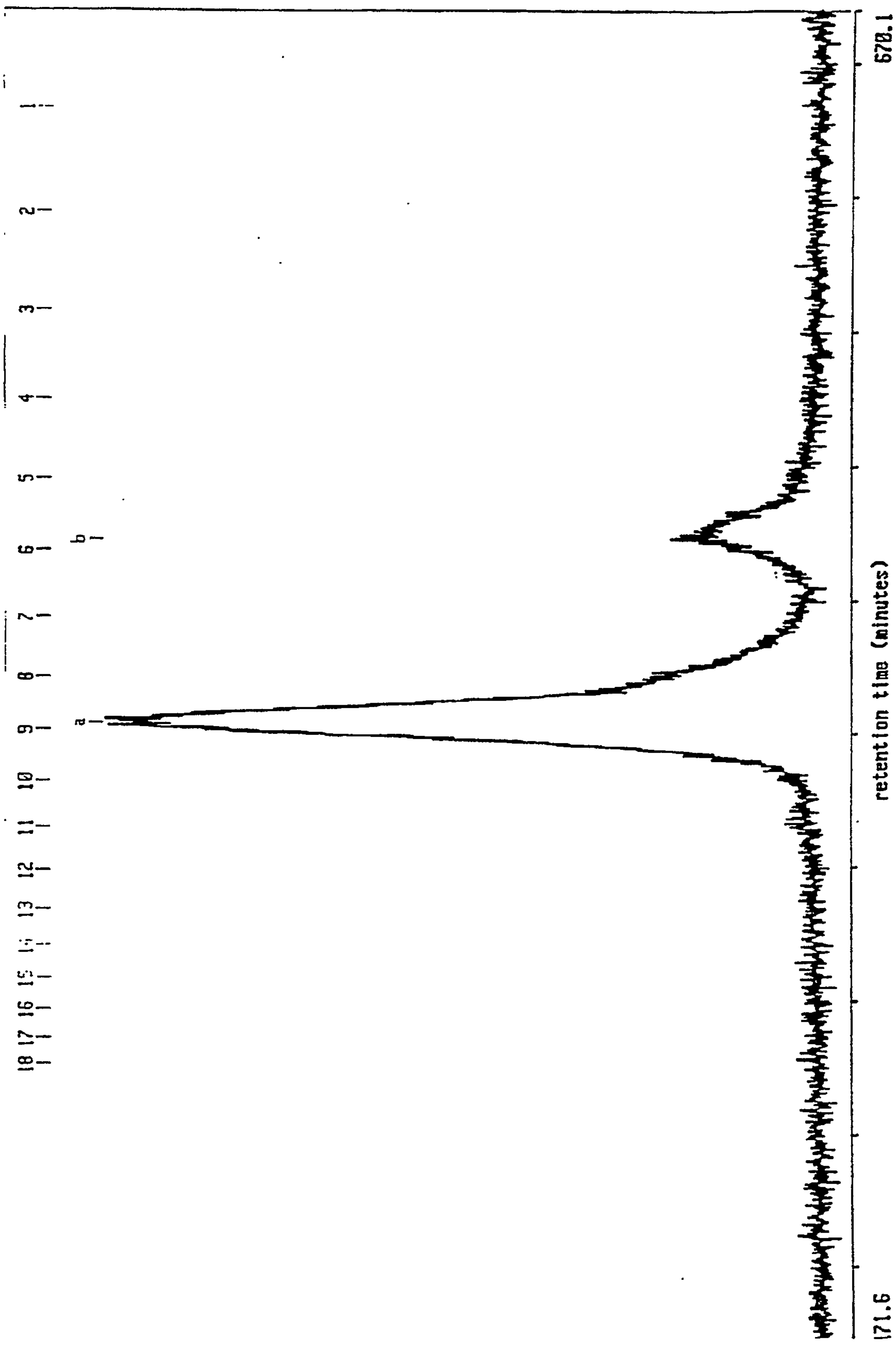

1
2
3
4
5
6
7
8
9
10
11
12
13
15
16
17
18
a
b
678.1
retention time (minutes)
171.6


Figure 4c

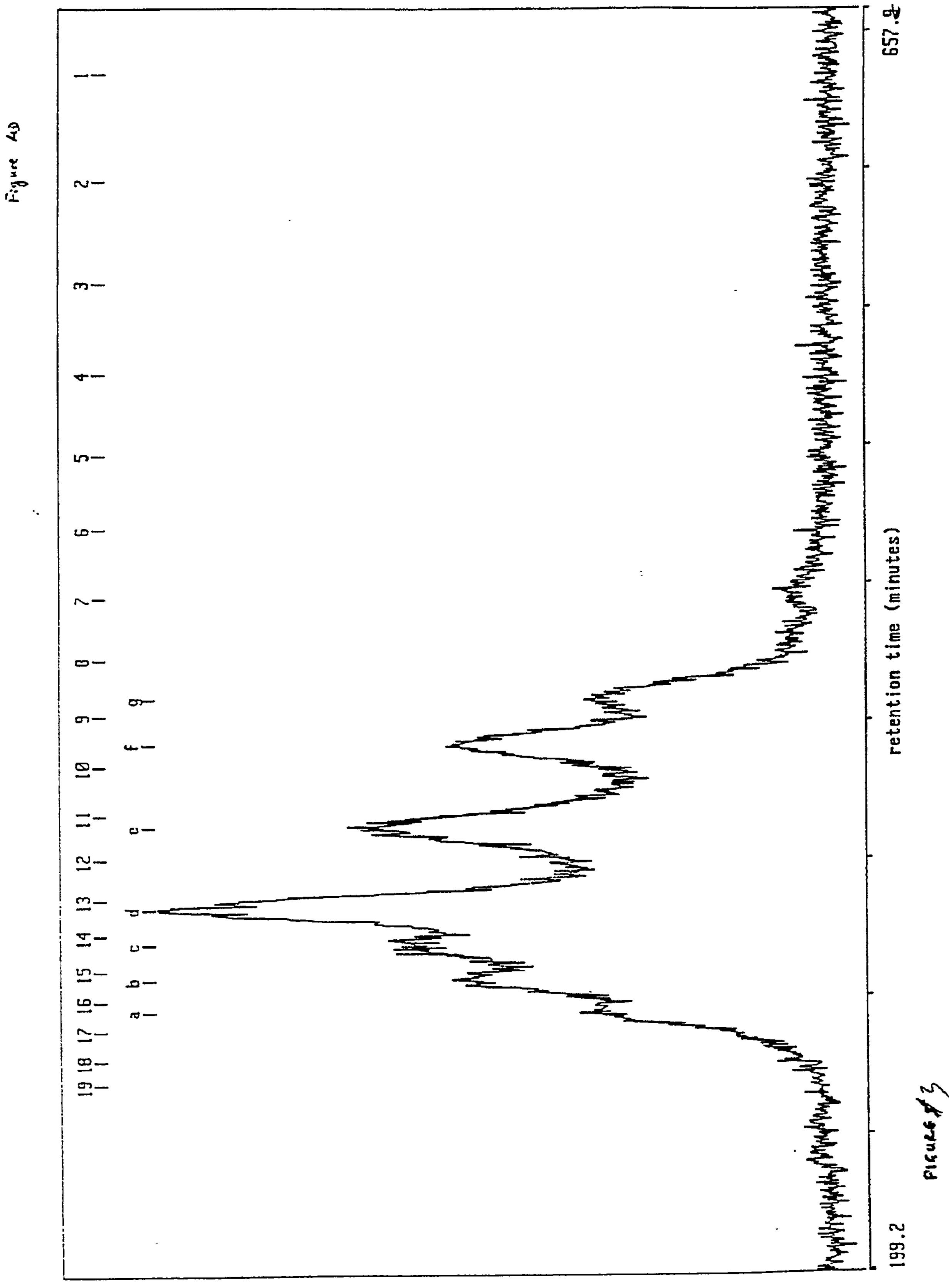
Figure 4D
1
2
3
4
5
6
7
8
9
10
11
12
13
14
15
16
17
18
19
a
b
c
d
e
f
g
657.9
199.2
retention time (minutes)

Oxford GlycoSystems

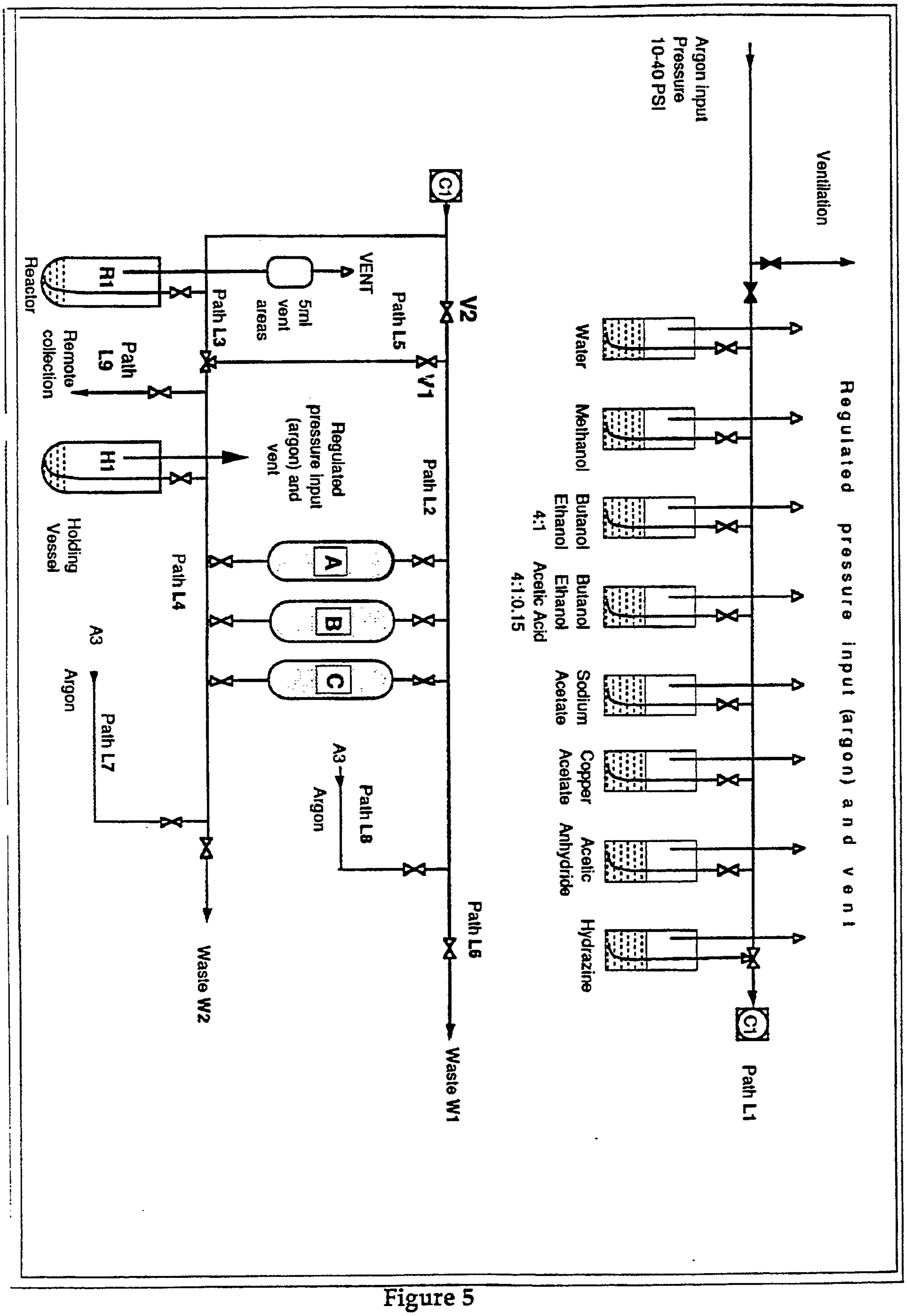


Figure 5